# EUROPEAN PATENT SPECIFICATION

(11) **EP 2 932 989 B1**
(45) Date of publication and mention of the grant of the patent: **05.06.2019**
(21) Application number: 13862143.8
(22) Date of filing: 09.01.2013
(51) Int. Cl.: A61L 33/12, A61K 38/36

(54) **TISSUE SEALANT IN WHICH COLLAGEN AND FIBRIN ARE MIXED, AND METHOD FOR PREPARING SAME**
GEWEBEKLEBSTOFF MIT EINER KOLLAGEN- UND FIBRINMISCHUNG UND VERFAHREN ZUR HERSTELLUNG DAVON
PRODUIT D'ÉTANCHÉITÉ DE TISSU, DANS LEQUEL SONT MÉLANGÉS DU COLLAGÈNE ET DE LA FIBRINE, ET SON PROCÉDÉ DE PRÉPARATION

(30) Priority: 11.12.2012 KR 20120143519
(43) Date of publication of application: 21.10.2015
(73) Proprietor: Sewoncellontec Co., Ltd., Seoul 150-724 (KR)
(72) Inventor: YOO, Ji Chul, Namyangju-si Gyeonggi-do 472-869 (KR); YEO, Se Ken, Yongin-si Gyeonggi-do 446-740 (KR); KIM, Jang Hoon, Seoul 135-231 (KR); LEE, Jun Keun, Seoul 131-220 (KR); SUH, Dong Sam, Seoul 139-924 (KR); CHANG, Cheong Ho, Seoul 135-841 (KR)
(74) Representative: dompatent von Kreisler Selting Werner - Partnerschaft von Patent- und Rechtsanwälten mbB
(86) International application number: PCT/KR2013/000143
(87) International publication number: WO 2014/092239

(56) References cited:
- EP-A2- 0 993 311
- EP-A2- 1 547 626
- KR-A- 20090 101 273
- KR-A- 20120 125 465
- KR-B1- 101 114 773
- US-A1- 2005 118 156
- US-A1- 2005 186 283
- US-A1- 2012 207 736

## Description

### Technical Field

The present invention relates to a composition in which collagen and fibrin are mixed, for use as a tissue sealant, and a method for preparing the same, and more specifically, the present invention is to supplement strength and degradability, which are indicated as weaknesses of a fibrin sealant in the market. That is, the present invention relates to a tissue sealant which, while having an affinity with cells, activates platelets contained in the blood to induce tissue regeneration, and thus, quality and reliability of products can be significantly improved to satisfy various needs of consumers who are users.

### Background Art

As is generally known, medical sealants have been applied to various fields ranging from surgical adhesion and attachment to hemostasis, and have a long history. Since a medical sealant material is directly applied to human tissues, a biocompatible material needs to be used. Since the medical sealant material may substantially flow into the body fluid or blood, the medical sealant material needs to be strictly biocompatible and biodegradable, can be sterilized, and should not exhibit toxicity and harm. In addition, it is important to select a sealant material that has a high affinity with biological tissues even after being applied into the tissues and thus does not interfere with the regeneration into original tissues.

Currently, cyanoacrylate, polyurethane, gelatin, fibrin, or the like, as a medical sealant material, is applied to products. Medical sealants have generally been used in several fields, such as skin, blood vessels, digestive organs, brain nerves, plastic surgery, orthopedics, and the like. Such sealants are required to have prompt adhesive strength in a moisture environment, be sterilizable and untoxic, and not interfere with sufficient mechanical properties, biodegradability, effective hemostasis, and body healing in view of the wound.

Cyanoacrylate is mainly used for industrial purposes, and is used in no more than 5% for a medical purpose. However, since cyanoacrylate has the possibility of substituting for a suture, studies thereof are actively conducting in, especially, developed countries. However, cyanoacrylate is vulnerable to impact, has deteriorations in heat resistance and water resistance after being applied, and retains toxicity and vulnerability to some tissues, and thus, cyanoacrylate is currently used restrictively.

Polyurethane is a material that keeps the flexibility of an attachment region, and has advantages in that polyurethane is fast hardened due to good reactivity with water, and the hardened material maintains its elasticity. On the other hand, polyurethane has a disadvantage in that aromatic diisocyanate, which is a synthetic raw material, is biologically toxic.

A glue using gelatin is a bio-derived sealant, and examples thereof are a product in which gelatin and resorcinol cross-link by formalin and a product using gelatin, polyglutamic acid, and carbodiimide. These products may exhibit toxicity using a chemical cross-linking agent of formalin and carbodiimide. Products that employ formalin as a cross-linking agent have been used in some countries, but the licensing thereof is under way and the effectiveness thereof are being tested in Japan and the like.

Fibrin glue is a product that is obtained by applying the principle of fibrin formation using fibrinogen, thrombin, calcium chloride, and the like as materials. Fibrin glue has rapid adhesion, requires no heat or pressure, is not significantly affected by the environment of a glued region, and retains biological advantages of being biocompatible and biodegradable. However, fibrin glue has disadvantages in that it lacks physical properties and has a relatively higher biodegradation rate when compared with a sealant using a synthetic material. In order to overcome such disadvantages, researches on the inhibition on fibrinolytic enzymes through the addition of aprotinin in order to slow down the degradation rate of a fibrin polymer and retain a shape are being conducted. The use of collagen as an additive to supplement such disadvantages will make a significant contribution to complementing a fibrin glue formulation.

Fibrin used for a fibrin glue is applied and commercialized as a natural adhesive or hemostat, and has biocompatibility and biodegradability. Fibrin is known to be generally absorbed in the procedure of wound healing within several weeks and to have no side effects, such as inflammation, immune responses, tissue necrosis, or fiber hypertrophy. In addition, fibrin is a natural support for fibroblasts, and plays an important role in wound healing. The concept of a fibrin product has been established in the 1970s. The first product has been commercialized in Europe in 1982, and then has been used up to now. Recently, fibrin has been verified as a support for biological tissue engineering in many studies, and has been applied in various fields, such as orthopedics, dentistry, and neurosurgery.

Collagen that can be used as an additive to supplement the disadvantages of the fibrin glue is a structural protein component. Collagen constitutes soft tissues, such as dermis, tendon/ligament, and blood vessel, and hard tissues, such as bone and cartilage, and accounts for approximately 1/3 of the whole-body protein content in mammals. More than twenty types of collagen have been known, and type I collagen constituting skin, tendon/ligament, bone, and the like accounts for approximately 90% of the collagen in the body. Collagen is the protein that is made up of three strands and has a molecular weight of 300,000 daltons (about 100,000 daltons for each strand). In collage, glycine, which is the smallest amino acid unit (having the smallest molecular weight), is repeatedly connected (-G X Y-; glycine is continuously repeated, and X and Y vary). Therefore, glycine accounts for about 1/3 of amino acids constituting collagen. Collagen has been currently used for the medical purpose in fields of hemostat, a wound covering agent, artificial blood vessels, wrinkle improvement, and the like. In cases of the hemostat, Aviten, which is a collagen powdered product extracted from the calf skin, was first developed in 1974, and has been used up to now.

Most of all, the most important characteristic of collagen used in the medical regeneration field is that collagen is a material that is biologically compatible in the human tissue to exhibit an affinity with cells, and thus is important in the adhesion and growth of cells and the maintenance of viability. In addition, collagen stimulates platelets contained in the blood to induce growth factors contained in the platelets, thereby regenerating damaged tissues. Also, collagen has a triple helix structure and its degradability can be relatively maintained compared with a single structure of protein, and thus collagen may serve as a scaffold in the body.

Such material binding can fundamentally retain biodegradable characteristics that the tissue sealant needs to have and maintain characteristics of not interfering with regeneration. In addition, such material binding can complement physical properties that the fibrin glue lacks, and slow down the degradation rate, thereby providing degradable regeneration bones. Thus, the tissue sealant will promote the tissue generation and accelerate the regeneration procedure to shorten the therapy process, in addition to a role as a simple sealant. In addition, in order to use the tissue sealant promptly, the tissue sealant may be a formulation that is mounted in a prefilled type and freeze-stored.

The tissue sealant is a product that reduces the burden of a patient on the surgical procedure and maximizes the satisfaction in cases where a wounding is sutured and coated, such as having a lower risk of pain and infection compared with the conventional methods and shortening the operation time. Therefore, the tissue sealant will be highly favored in this field, and the market scale will be gradually expanded. Further, such a product helps the generation of tissues, and is used for a drug delivery system and a scaffold for regeneration, and thus contributing to a regenerative medical field.

US 2012/0207736 A1 discloses a composition for cartilaginous tissue repair produced by dissolving freeze-dried fibrinogen in an aprotinin solution, dissolving freeze-dried thrombin in a stabilizing solution, mixing an enriched collagen solution with thrombin and the stabilizing solution and installing the fibrinogen solution to one side of a dual kit and the solution containing the collagen to the other side, subsequently mixing the two compositions and injecting into damaged cartilaginous tissue.

US 2005/0118156 A1 discloses a ready-to-use, instantly available fibrin sealant composition prepared from a storage stable, aqueous fibrinogen solution component and an activated thrombin or thrombin-like component.

EP 099311 A2 discloses a hemostatic composition comprising thrombin and microfibrillar collagen in an aqueous medium.

### Detailed Description of the Invention

### Technical Problem

(Patent document 1) Korean patent publication No. 2012-0125465 (patent application No. 2012-7018109, title: dry powder fibrin sealant) has been filed.

Therefore, the present invention has been made in view of the above-mentioned problems, and the present invention provides a tissue sealant which collagen and fibrin are mixed and a method for preparing the same, wherein a first purpose of the present invention is to include the steps of: mixing a first material using fibrinogen and aprotinin; mixing a second material using thrombin, calcium chloride, and collagen; and mixing the first material and the second material with each other to prepare a third material; according to a second purpose of the present invention, it was verified that, as a result of comparison of physical strength, a sealant containing collagen showed high strength; according to a third purpose of the present invention, it was verified that, as a result of long-term/short-term degradability testing, a sealant containing collagen showed low degradability; according to a fourth purpose of the present invention, it was verified that, through electron microscopic observation, collagen and fibrinogen are combined to show a stable structure; according to a fifth purpose of the present invention, it was verified that, as a result of comparison of growth and viability using chondrocytes, osteoblasts, and adipose-derived cells, a structure containing collagen showed a good growth rate and high viability; according to a sixth purpose of the present invention, it was verified that the inclusion of collagen maintains high strength and a stable structure and supplies a material having affinities with cells/blood, thereby greatly helping the regeneration of deleted/damaged region; a seventh purpose of the present invention is to activate platelets included in the blood to induce the tissue regeneration; and an eighth purpose of the present invention is to significantly improve quality and reliability of products, thereby satisfying various needs of consumers who are users, thus giving a good impression.

### Technical Solution

In accordance with an aspect of the present invention, there is provided a method for preparing a tissue sealant as defined in claim 2.

In accordance with another aspect of the present invention, there is provided a tissue sealant as defined in claim 1.

A preferred embodiment of the present invention is reflected in claim 3.

The scope of the invention is defined by the claims. Any references in the description to methods of treatment refer to the products of the present invention for use in a method for treatment.

### Advantageous Effects

As set forth above, the present invention includes the steps of: mixing a first material using fibrinogen and aprotinin; mixing a second material using thrombin, calcium chloride, and collagen; and mixing the first material and the second material with each other to prepare a third material.

According to the present invention having the foregoing technical feature, it was verified that, as a result of comparison of physical strength, a sealant containing collagen showed high strength.

In addition, according to the present invention, it was verified that, as a result of long-term/short-term degradability testing, a sealant containing collagen showed low degradability.

In addition, according to the present invention, it was verified that, through electron microscopic observation, collagen and fibrinogen are combined to show a stable structure.

In addition, according to the present invention, it was verified that, as a result of comparison of growth and viability using chondrocytes, osteoblasts, and adipose-derived cells, a structure containing collagen showed a good growth rate and high viability.

In addition, according to the present invention, it was verified that the inclusion of collagen maintains high strength and a stable structure and supplies a material having affinities with cells/blood, thereby greatly helping the generation of deleted/damaged region.

In addition, the present invention is to activate platelets included in the blood to induce the tissue regeneration.

The present invention can significantly improve quality and reliability of products through the foregoing effects, thereby satisfying various needs of consumers as users thereof, thus giving a good impression, and thus the present invention is very useful.

Hereinafter, preferred embodiments of the present invention for attaining the above effects will be described in detail with reference to the accompanying drawings.

### Brief Description of the Drawings

FIG. 1 is an electron micrographic image of a tissue sealant in which collage and fibrin are mixed according to the present invention (20,000 X, critical point drying).
FIG. 2 is a conceptual diagram showing the binding of collagen and a cell.
FIG. 3 is a conceptual diagram showing the activation of platelets in collagen.
FIG. 4 is a comparison graph showing the degradation rates of tissue sealants in which collagen and fibrin are mixed.
FIG. 5 is a graph showing the proliferation rates of chondrocytes in tissue sealants in which collagen and fibrin are mixed
FIG. 6 illustrates images confirming the proliferation and viability of chondrocytes in tissue sealants in which collagen and fibrin are mixed according to the present invention.
FIG. 7 is a graph showing the proliferation rates of osteoblasts in tissue sealants in which collagen and fibrin are mixed
FIG. 8 illustrates images confirming the proliferation and viability of osteoblasts in tissue sealants in which collagen and fibrin are mixed according to the present invention.
FIG. 9 illustrates images confirming the proliferation and viability of adipose-derived cells in tissue sealants in which collagen and fibrin are mixed according to the present invention.
FIG. 10 is a diagram of a state in which a tissue sealant in which collagen and fibrin are mixed according to the present invention is loaded in a two-way syringe.

### Mode for Carrying Out the Invention

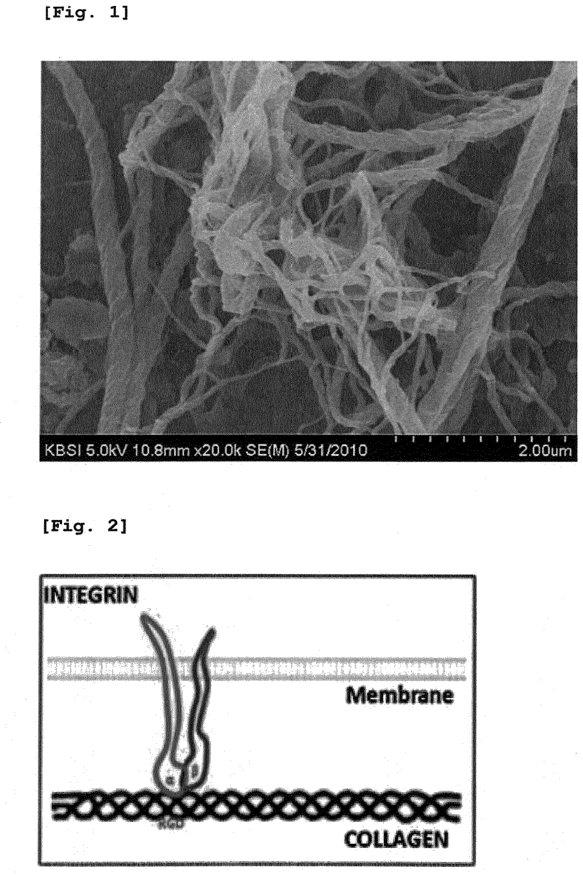

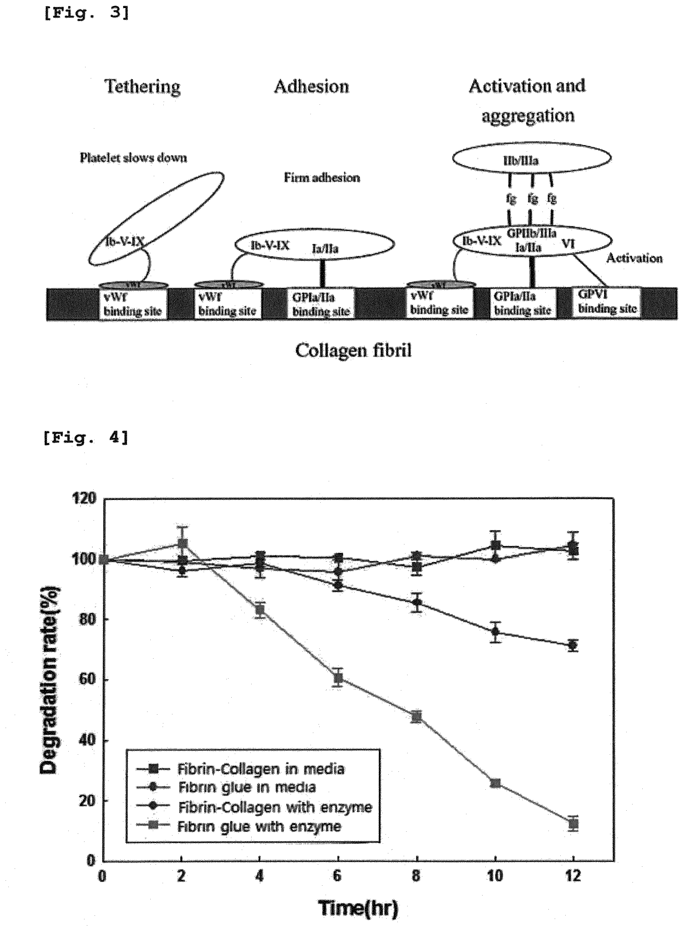

A tissue sealant in which collagen and fibrin are mixed and a method for preparing the same according to the present invention are as shown in FIGS. 1 to 10.

In the following descriptions, when it is determined that detailed descriptions of known functions or constitutions associated with the present invention obscure the gist of the present invention, detailed descriptions thereof will be omitted.

In addition, the terms to be later described are defined in consideration of functions in the present invention, and thus the definitions of the terms are to be interpreted throughout the present specification since the terms may be interpreted by the intention of the producer or custom.

First, the present invention includes a step of mixing a first material using fibrinogen and aprotinin.

In addition, the present invention includes a step of mixing a second material using thrombin, calcium chloride, and collagen.

In addition, the present invention includes a step of mixing the first material and the second material with each other to prepare a third material, and thus a tissue sealant in which collagen and fibrinogen are mixed is prepared.

According to the present invention, the fibrinogen has a concentration of 65-130 mg/mL and the aprotinin has a concentration of 1,000-3,000 KIU/mL.

The thrombin has a concentration of 40-600 U/ml, the calcium chloride has a concentration of 4-140 mmol/L.

Less than 65 mg/mL of fibrinogen weakens physical strength, and more than 130 mg/mL of fibrinogen leads to the densification of the physical structure, resulting in reducing the pore sizes, thereby inhibiting cellular activity, and thus the concentration of fibrinogen is 65-130 mg/mL.

In addition, the concentration of the aprotinin is 1,000-3,000 KIU/mL. Less than 1,000 KIU/mL of aprotinin accelerates the degradation of a composition, and more than 3,000 KIU/mL of aprotinin increases the risk of causing anaphylaxis, and thus the concentration of aprotinin is 1,000-3,000 KIU/mL.

In addition, the concentration of the thrombin is 40-600 KIU/mL. Less than 40 U/ml of thrombin weakens the physical strength of a composition, and more than 60 U/ml of thrombin leads to the densification of the structure of the composition, which therefore has no affinity with cells and rapidly increases the gelation rate, failing to serve as a sealant in an applied region, and thus, the concentration of the thrombin is 40-60 U/ml.

In addition, the concentration of calcium chloride is 4-140 mmol/L. Less than 4 mmol/L of calcium chloride too slows down the gelation rate, and more than 140 mmol/L of calcium chloride may have a bad influence on cells due to a high osmotic pressure, and thus the concentration of calcium chloride is 4-140 mmol/L.

The concentration of the collagen is 10-30 mg/mL.

That is, less than 10 mg/mL of collagen weakens physical strength, and more than 30 mg/mL has a bad influence on degradability and a stable structure and has no affinity with cells and blood, and thus the concentration of the collagen is 10-30 mg/mL.

Meanwhile, the method for preparing a tissue sealant in which collagen and fibrin are mixed according to the present invention will be specifically described as follows.

First, a step of preparing a first material including fibrinogen and aprotinin is conducted.

After that, a step of preparing a second material including thrombin, calcium chloride, and collagen is conducted.

Then, a step of putting the first material in one side of a two-way syringe and the second material in the other side of the two-way syringe and then mixing the first and second materials with each other is conducted, and thus, a tissue sealant in which collagen and fibrin are mixed is prepared.

According to the present invention, aprotinin and calcium solutions are injected into the fibrinogen and the thrombin, respectively, and the thrombin is mixed with a collagen solution, and then the resultant solutions are loaded in the two-way syringe, thereby preparing a tissue sealant in which collagen and fibrin are mixed.

According to the present invention, a tissue sealant in which collagen and fibrin are mixed can be prepared by going through the respective steps for preparing a tissue sealant in which collagen and fibrin are mixed.

The tissue sealant in which collagen and fibrin are mixed and the method for preparing the same according to the present invention will be described by giving examples.

### (Example 1)

Comparison of physical properties between present invention and prior art

In order to verify physical properties of the present invention, the maximum stress, the gel strength, and the tensile strength were checked using a physical property meter.

### 1. Sample preparation

1) In the prior art, the Greenplast product was used (comparative example).
2) For components of the present invention, dried fibrinogen and thrombin of Greenplast were dissolved in an aprotinin solution and a calcium solution added thereto, respectively. Here, the thrombin solution was mixed with a 3% collagen solution. The resultant solutions were loaded in a two-way syringe.
3) For the measurement of physical properties, each sample was put in a cylindrical-shaped mold (Φ12 X 15 mm) to manufacture a form.

### 2. Measurement of physical properties

1) Physical property meter: Rheometer (CR-500DX, Sun scienctific rheometer)
2) Test items: maximum stress (N), gel strength (g-cm), tensile strength (g/cm2)
3) Test conditions: entry distance (7.5 mm), table speed (50 mm/min), maximum stress (10 kg), adapter (No.1 Φ20mm)

### 3. Test results

**[Table 1]**

| Classification | Maximum stress N | Gel strength g-cm | Tensile strength g/cm2 |
|---|---|---|---|
| Present invention | 19.3 | 1212.9 | 2493.7 |
| Prior art | 13.4 | 831.7 | 1692.2 |

### (Example 2)

### Comparison of degradability between present invention and prior art product (short-term/long-term)

In order to verify degradability of the composition of the present invention, the degradability of the fibrin glue product and the material for a predetermined period was checked.

### 1. Degradability (short-term)

### 1) Sample preparation

- In the prior art, the Greenplast product was used (comparative example).
- For components of the present invention, dried fibrinogen and thrombin of Greenplast were dissolved in an aprotinin solution and a calcium solution added thereto, respectively. Here, the thrombin solution was mixed with a 3% collagen solution. The resultant solutions were loaded in a two-way syringe.
- For the measurement of physical properties, each sample was put in a cylindrical-shaped mold (Φ8 X 5 mm) to manufacture a form.

### 2) Treatment conditions for degradability verification

- Two conditions were confirmed for the solvent. A condition of using only a DEME medium and a condition of using a DMEM medium containing Liberase TM were confirmed (the concentration of Liberase TM was set to 10 ug/mL).
- The sample was put in a 12-well plate, and the degradation aspect was checked as a residue weight of the composition in the 2-hour unit for 12 hours.

### 3) Test results

- It was verified that, in the condition of degradation through the enzyme treatment, over 90% of the formulation of the prior art was degraded within 12 hours, and about 80% of the composition of the present invention remained for 12 hours. In the DMED condition, the degradation was not verified for 12 hours.

### 2. Degradability (long-term)

### 1) Sample preparation

- In the prior art, the Greenplast product was used (comparative example).
- For components of the present invention, dried fibrinogen and thrombin of Greenplast were dissolved in an aprotinin solution and a calcium solution added thereto, respectively. Here, the thrombin solution was mixed with a 3% collagen solution. The resultant solutions were loaded in a two-way syringe.
- For the measurement of physical properties, each sample was put in a cylindrical-shaped mold (Φ12 X 15 mm) to manufacture a form.

### 2) Treatment conditions for degradability verification

- DMEM was used for the solvent, and the naked eye observation was conducted at 37°C for one month.

### 3) Test results

- The composition of the present invention was observed for one month or longer, but the formulation of the prior art was degraded within three weeks. It was verified that the degradation period of the composition of the present invention was longer than that of the formulation of the prior art.

### (Example 3)

### Electron micrographic analysis of present invention

The structure of the composition of the present invention was observed by an electron microscope.

### 1. Sample preparation

- For the preparation of the composition, a fibrinogen solution of Greenplast and a collagen-containing thrombin solution/calcium solution were prepared. The concentration of the collagen solution was 3% (w/v).
- Each of the prepared solutions was applied to a two-way syringe to be dispensed on trays for electron microscopic observation, and then gelated.

### 2. Methods

- The composition of the present invention was dried at the critical point, and then observed by an electron microscope.
- The critical point drying of the composition was conducted through alcohol treatment in a critical point drier (Hitachi, HCP-2).
- The sample for electron microscopic observation was cut and gold-coated, and then observed using SEM (Hitachi, S3500).
- The electron microscopic analysis was conducted at the magnification of 20,000.

### 3. Test results

As a result of observing in the electron microscope at the magnification of 20,000, it was verified that collagen and fibrin cross-linked with each other. The fibrous structure of collagen was also observed. It can be anticipated that such a material cross-linkage enhances physical properties.

### (Example 4)

### Test on cell compatibility and growth of present invention (chondrocytes)

In order to verify proliferation and viability of chondrocytes in the composition of the present invention, CCK-8 assay and Calcein-AM & EthD-1 staining were used.

### 1. Cells and composition

- Chondrocytes of animals excluding human were used. The number of cells was 12,000,000 in the composition mixture liquid.
- For the composition, Greenplast, which is the fibrin glue product, was mixed with 3% (according to the invention) and 6% (comparative example) of collagen, respectively.
- The composition was prepared by dissolving dried fibrinogen of Greenplast in 1 mL of a solution containing chondrocytes and mixing the thrombin/calcium solution with 1 mL of a collagen solution with each concentration.
- The fibrinogen solution containing chondrocytes and the thrombin solution containing collagen were used to prepare a total of 2 mL of collagen-fibrin solution.
- For culture, the prepared collagen-fibrin component was dispensed in a 24-well plate at 0.2 mL for each well, and observation was conducted for 20 days while the DMEM medium was exchanged every 2-3 days.

### 2. CCK-8 assay

- The medium was removed from each well (24-well plate) containing a cultured material.
- 1 mL of a new medium was put in each well.
- A CCK-8 (Dojindo, CK04-11) reagent was added in 100 uL for each well, which corresponds to 10% of the volume of the medium, followed by a reaction in 5% CO2 incubator at 37°C for 3 hours.
- Upon completion of the reaction, the absorbance (450 nm) of the reaction liquid was read using a microplate reader.
- The cell proliferation for each culture period was verified using the measured OD value.

### 3. Calcein-AM & EthD-1 staining

- A working solution was prepared by mixing a buffer solution with 2 µM and 4 µM of Calcein AM and EthD-1 of the LIVE/DEADViability/Cytotoxicity Assay Kit (Invitrogen, L3224), respectively.
- The cultured material was transferred to a new 24-well plate, and then 1 mL of the working solution was put therein, followed by a reaction in the condition where the light is blocked for 20 minutes. After that, live cells and dead cells were observed using a fluorescent microscope. The live cells were observed as green and the dead cells were observed as red.

### 4. Test results

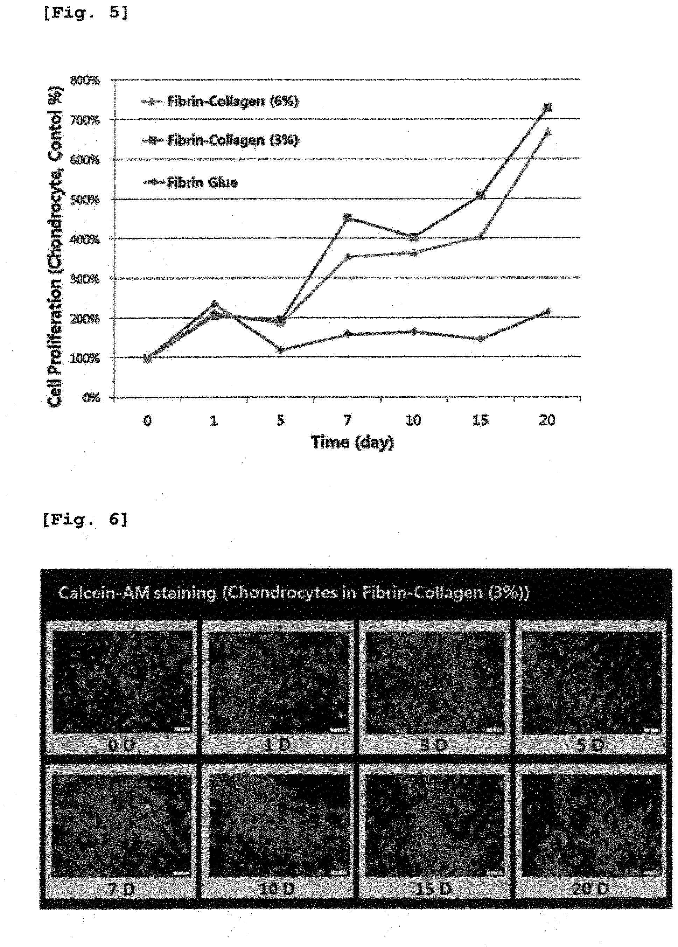

A. CCK-8 assay (FIG. 5 in which Fibrin and Fibrin-Collagen 6% are comparative examples and Fibrin-Collagen 3% is an example according to the present invention)
B. Calcein-AM & EthD-1 staining (FIG. 6)

### (Example 5)

### Test on cell biocompatibility of present invention (osteoblasts)

In order to verify proliferation and viability of osteoblasts in the composition of the present invention, CCK-8 assay and Calcein-AM & EthD-1 staining were used.

### 1. Cells and composition

- Osteoblasts of animals excluding human were used. The number of cells was 12,000,000 in the composition mixture liquid.
- The composition was configured by mixing Greenplast, which is the fibrin glue product, with 3% (according to the invention) and 6% (comparative example) of collagen, respectively.
- The composition was prepared by dissolving dried fibrinogen of Greenplast in 1 mL of a solution containing chondrocytes and mixing the thrombin/calcium solution with 1 mL of a collagen solution with each concentration.
- The fibrinogen solution containing osteoblasts and the thrombin solution containing collagen were use to prepare a total of 2 mL of collagen-fibrin solution.
- For culture, the prepared collagen-fibrin component was dispensed in a 24-well plate at 0.2 mL for each well, and observation was conducted for 20 days while the α-DMEM medium was exchanged every 2-3 days.

### 2. CCK-8 assay

- The medium containing cultured material was removed from each well (24-well plate).
- 1 mL of a new medium was put in each well.
- A CCK-8 (Dojindo, CK04-11) reagent was added in 100 uL for each well, which corresponds to 10% of the volume of the medium, followed by a reaction in 5% CO2 incubator at 37°C for 3 hours.
- Upon completion of the reaction, the absorbance (450 nm) of the reaction liquid was read using a microplate reader.
- The cell proliferation for each culture period was verified using the measured OD value.

### 3. Calcein-AM & EthD-1 staining

- A working solution was prepared by mixing a buffer solution with 2 µM and 4 µM of Calcein AM and EthD-1 of the LIVE/DEADViability/Cytotoxicity Assay Kit (Invitrogen, L3224), respectively.
- The cultured material was transferred to a new 24-well plate, and then 1 mL of the working solution was put therein, followed by a reaction in the condition where the light is blocked for 20 minutes. After that, live cells and dead cells were observed using a fluorescent microscope.

### 4. Test results

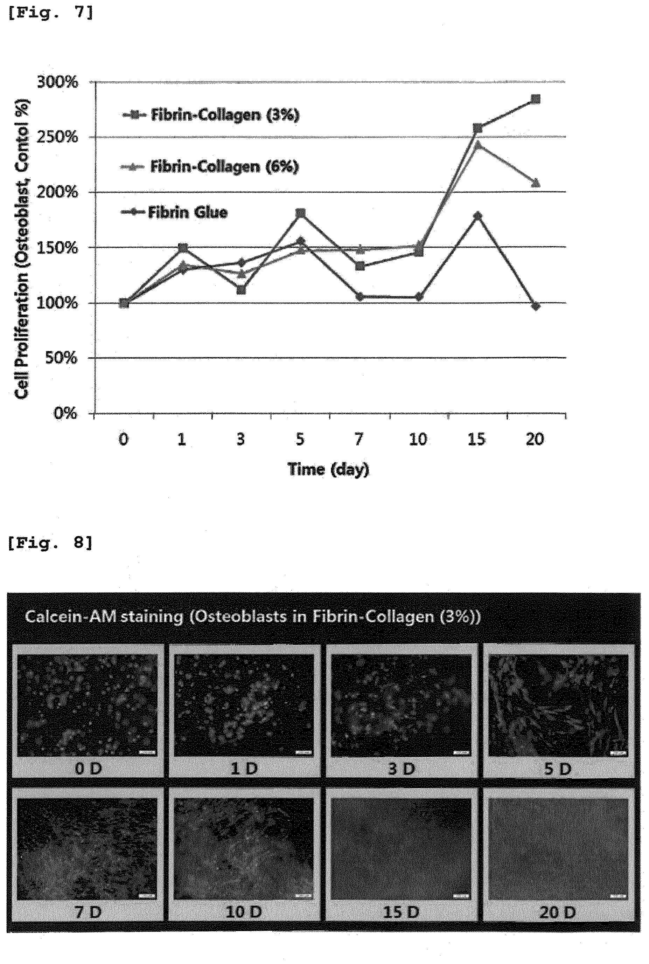

A. CCK-8 assay (FIG. 7 in which Fibrin and Fibrin-Collagen 6% are comparative examples and Fibrin-Collagen 3% is an example according to the present invention)
B. Calcein-AM & EthD-1 staining (FIG. 8)

### (Example 6)

### Test on cell biocompatibility of present invention (adipose-derived cells)

In order to verify proliferation and viability of adipose-derived cells in the composition of the present invention, CCK-8 assay and Calcein-AM & EthD-1 staining were used.

### 1. Cells and composition

- Adipose-derived cells were used. The number of cells was 12,000,000 in the composition mixture liquid.
- The composition was configured by mixing Greenplast, which is the fibrin glue product, with 3% of collagen, respectively.
- The composition was prepared by dissolving dried fibrinogen of Greenplast in 1 mL of a solution containing adipose-derived cells and mixing the thrombin/calcium solution with 1 mL of a collagen solution with each concentration.
- The fibrinogen solution containing adipose-derived cells and the thrombin solution containing collagen were used to prepare a total of 2 mL of collagen-fibrin solution.
- For culture, the prepared collagen-fibrin component was dispensed in a 24-well plate at 0.2 mL for each well, and observation was conducted for 20 days while the DMEM medium was exchanged every 2-3 days.

### 2. Calcein-AM & EthD-1 staining

- A working solution was prepared by mixing PBS with 2 µM and 4 µM of Calcein AM and EthD-1 of the LIVE/DEADViability/Cytotoxicity Assay Kit (Invitrogen, L3224), respectively.
- The cultured material was transferred to a new 24-well plate, and then 1 mL of the working solution was put therein, followed by a reaction in the condition where the light is blocked for 20 minutes. After that, live cells and dead cells were observed using a fluorescent microscope.

### 3. Test results

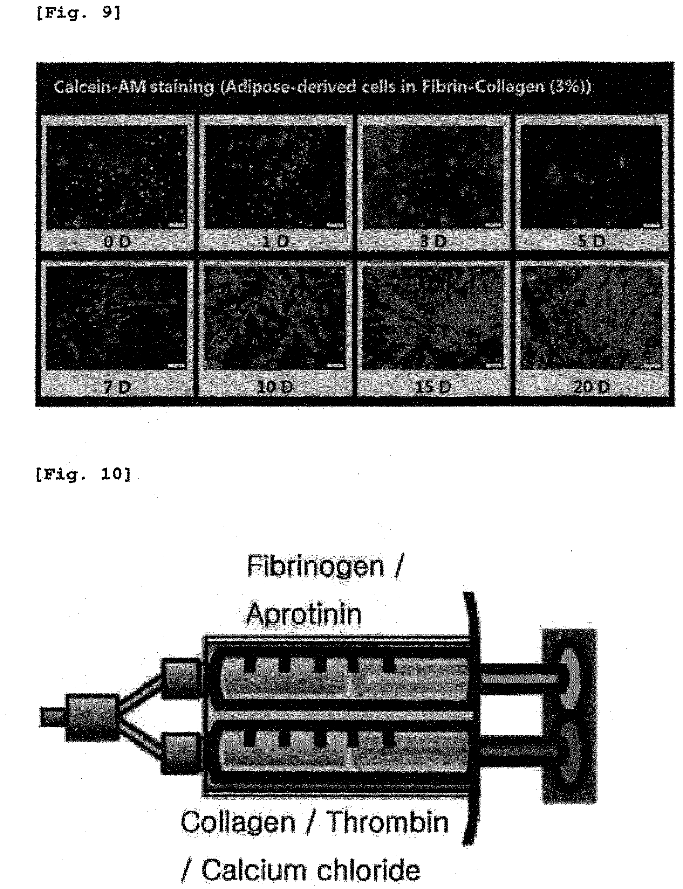

- Calcein-AM & EthD-1 staining (FIG. 9)

## Claims

1. A composition, in which collagen and fibrin are mixed, for use as a tissue sealant, wherein the composition is prepared by
mixing fibrinogen and aprotinin to prepare a first material;
mixing thrombin, calcium chloride, and collagen to prepare a second material; and
mixing the first material and the second material with each other to prepare a third material, and
wherein the fibrinogen has a concentration of 65-130 mg/mL, the aprotinin has a concentration of 1,000-3,000 KIU/mL,
the thrombin has a concentration of 40-600 U/ml,
the calcium chloride has a concentration of 4-140 mmol/L,
and the collagen has a concentration of 10-30 mg/mL.

2. A method for preparing a composition for use as a tissue sealant, in which collagen and fibrin are mixed, wherein the method comprises:
preparing a first material including fibrinogen and aprotinin;
preparing a second material including thrombin, calcium chloride, and collagen; and
putting the first material in one side of a two-way syringe and the second material in the other side of the two-way syringe, and then mixing the first and second materials with each other, and
wherein the fibrinogen has a concentration of 65-130 mg/mL, the aprotinin has a concentration of 1,000-3,000 KIU/mL, the thrombin has a concentration of 40-600 U/ml, the calcium chloride has a concentration of 4-140 mmol/L, and the collagen has a concentration of 10-30 mg/mL.

3. A method according to claim 2, wherein aprotinin and calcium solutions are injected into the fibrinogen and the thrombin, respectively, and the thrombin is mixed with a collagen solution, and then the resultant solutions were loaded in the two-way syringe.

## Patentansprüche

1. Zusammensetzung, bei der Collagen und Fibrin miteinander gemischt sind, zur Verwendung als Gewebedichtungsmittel, wobei die Zusammensetzung hergestellt wird durch
Mischen von Fibrinogen und Aprotinin zur Herstellung eines ersten Materials;
Mischen von Thrombin, Calciumchlorid und Collagen zur Herstellung eines zweiten Materials; und
Mischen des ersten Materials mit dem zweiten Material zur Herstellung eines dritten Materials; und
wobei das Fibrinogen eine Konzentration von 65-130 mg/ml aufweist, das Aprotinin eine Konzentration von 1000-3000 kIE/ml aufweist,
das Thrombin eine Konzentration von 40-600 E/ml aufweist,
das Calciumchlorid eine Konzentration von 4-140 mmol/l aufweist
und das Collagen eine Konzentration von 10-30 mg/ml aufweist.

2. Verfahren zur Herstellung einer Zusammensetzung zur Verwendung als Gewebedichtungsmittel, bei der Collagen und Fibrin miteinander gemischt sind, wobei das Verfahren Folgendes umfasst:
Herstellen eines ersten Materials, das Fibrinogen und Aprotinin umfasst;
Herstellen eines zweiten Materials, das Thrombin, Calciumchlorid und Collagen umfasst; und
Einfüllen des ersten Materials auf einer Seite einer Zweiwegespritze und des zweiten Materials auf der anderen Seite der Zweiwegespritze und dann Mischen des ersten Materials mit dem zweiten Material; und
wobei das Fibrinogen eine Konzentration von 65-130 mg/ml aufweist, das Aprotinin eine Konzentration von 1000-3000 kIE/ml aufweist, das Thrombin eine Konzentration von 40-600 E/ml aufweist, das Calciumchlorid eine Konzentration von 4-140 mmol/l aufweist und das Collagen eine Konzentration von 10-30 mg/ml aufweist.

3. Verfahren gemäß Anspruch 2, wobei eine Aprotininlösung in das Fibrinogen und eine Calciumlösung in das Thrombin injiziert werden und das Thrombin mit einer Collagenlösung gemischt wird und dann die resultierenden Lösungen in die Zweiwegespritze eingefüllt werden.

## Revendications

1. Composition dans laquelle du collagène et de la fibrine ont été mélangés pour être utilisée comme agent de scellement tissulaire, dans laquelle la composition est préparée par les étapes consistant à :
mélanger du fibrinogène et de l'aprotinine pour préparer une première matière,
mélanger de la thrombine, du chlorure de calcium et du collagène pour préparer une deuxième matière, et
mélanger ladite première matière et ladite deuxième matière ensemble pour préparer une troisième matière, et
dans laquelle le fibrinogène présente une concentration de 65-130 mg/ml, l'aprotinine présente une concentration de 1000-3000 kUI/ml,
la thrombine présente une concentration de 40-600 E/ml,
le chlorure de calcium présente une concentration de 4-140 mmol/l, et
le collagène présente une concentration de 10-30 mg/ml.

2. Procédé pour préparer une composition pour être utilisée comme agent de scellement tissulaire, dans laquelle du collagène et de la fibrine ont été mélangés, dans laquelle ledit procédé comprend les étapes consistant à :
préparer une première matière comprenant du fibrinogène et de l'aprotinine,
préparer une deuxième matière comprenant de la thrombine, du chlorure de calcium et du collagène, et
placer la première matière dans un côté d'une seringue à deux voies et placer la deuxième matière dans l'autre côté de la seringue à deux voies, et ensuite mélanger lesdites première et deuxième matières ensemble, et
dans laquelle le fibrinogène présente une concentration de 65-130 mg/ml, l'aprotinine présente une concentration de 1000-3000 kUI/ml, la thrombine présente une concentration de 40-600 E/ml, le chlorure de calcium présente une concentration de 4-140 mmol/l, et le collagène présente une concentration de 10-30 mg/ml.

3. Procédé selon la revendication 2, dans laquelle une solution d'aprotinine est injectée dans le fibrinogène et une solution de calcium est injectée dans la thrombine, et la thrombine est mélangée avec une solution de collagène, et ensuite les solutions obtenues sont chargées dans la seringue à deux voies.
